# EUROPEAN PATENT APPLICATION

(11) **EP 1 997 480 A1**
(43) Date of publication of application: **03.12.2008**
(21) Application number: 07010848.5
(22) Date of filing: 01.06.2007
(51) Int. Cl.: A61K 9/14

(54) **Mineral-fiber solid dispersion, method for preparing the same and use thereof as pharmaceutical tableting aid**

(71) Applicant: The Jordanian Pharmaceutical Manufacturing Co., 11710 Naor (JO)
(72) Inventor: Badwan, Adnan, Amman, 11185 (JO); Al-Remawi, Mayyas, Russiefa 13713 (JO); Said Rashid, Iyad, Amman 11151 (JO)
(74) Representative: Winkler, Andreas Fritz Ernst

(57) **Abstract**

A mineral-fiber solid dispersion comprising at least one hydrophilic polymeric material selected from the group consisting of hydrophilic polysaccharides having the ability to form a gel in water with minimal dissolution nature, and at least one silica-based trivalent or divalent salt of a water-absorbable compound selected from the group consisting of silicon dioxide, derivatives thereof or any mixtures thereof is described in this invention.

Also a method for preparing the mineral-fiber solid dispersion, wherein the method in conducted via a solid-in-solid dispersion technique which comprises mixing the fiber material with the water-absorbable compound in a presence of water and allowing the absorption of the water-absorbable compound in an appropriate ratio in the intimate structure of the fiber material, and next adjusting the process conditions in order to allow the precipitation of the water-absorbable compound into the mineral solid within the structure of the fiber material to form a solid-in-solid dispersion of mineral and fiber is described.

The mineral-fiber solid dispersion is used for manufacturing of multifunctional pharmaceutical filler or direct compressible excipient that can be used as a pharmaceutical tableting aid possessing powerful compaction, disintegration and flow properties.

## Description

### Field of the invention

The present invention relates to a novel pharmaceutical excipient composition, a method for preparing the same as well as use of a novel mineral-fiber solid dispersion as a direct compressible pharmaceutical filler with excellent flow, super-compaction, and superdisintegration properties for use in pharmaceutical solid dosage forms.

### Background of the invention

The present invention relates to a multi-functional pharmaceutical excipient that can be used during direct compression of active pharmaceutical ingredients (API).

The term "direct compression" is used to define the process by which tablets are compressed directly from the powder blends of active ingredients and suitable excipients. No pretreatment of the powder blends by wet granulation or dry granulation is involved.

The International Pharmaceutical Excipient Council (IPEC) defines excipients as "substances, other than the API in a finished dosage form, which have been appropriately evaluated for safety and are included in a drug delivery system to either aid the processing or to aid manufacture, protect, support, enhance stability, bioavailability or patient acceptability, assist in product identification, or enhance any other attributes of the overall safety and effectiveness of the drug delivery system or use".

The most important characteristics for a directly compressible excipient are summarized as follows:

The direct compressible excipient should be free flowing. Since flowability is required in case of high-speed tableting machines.

Compressibility is required for satisfactory tableting. Few excipients can be compressed directly without elastic recovery.

Dilution capacity can be defined as the amount of an active ingredient that can be satisfactorily compressed into tablets with the given excipient. A high dilution capacity is required for a directly compressible excipient. Thus, the final dosage form has a minimal possible weight.

The excipient should not exhibit any physical or chemical change on ageing and should be stable to air, moisture and heat.

The excipient should be capable of being reworked without loss of flow or compressibility.

The excipient should have particle size equivalent to the active material present in the formulation. Uniform and reproducible particle size is necessary to avoid segregation.

The excipient should be compatible with all active ingredients, physiologically inert, colorless, tasteless, relatively cost effective, available, with low lubricant sensitivity, and has a good batch-to-batch reproducibility.

There are many attempts for the formulation of directly compressible materials in the prior art [M.C. Gohel, A review of co-processed directly compressible excipients, J. Pharmaceut. Sci., 8 (1): 76-93, 2005].

For example, there are known direct compressible (DC) materials comprising lactose, cellulose derivative such as microcrystalline cellulose, sugars such as sucrose and starch.

Co-processing is an important methodology for the development of DC materials. Co-processing is a method used for the preparation of two or more excipients by an appropriate process. Co-processing could lead to the formation of excipients with superior properties compared to simple physical mixtures of their components. The main aim of co-processing is to obtain a product with added value related to the ratio of its functionality/price. Co-processing is interesting because the products are physically modified in a special way without altering the chemical structure. Examples on co-processed materials that are acceptable by the USP/NF are spray-crystallized dextrose-maltose (Emdex), and compressible sugars.

Other directly compressible excipients are: Cellactose (microcrystalline cellulose MCC, Lactose), XliTab (Xylitol, Na carboxymethylcellulose CMC), Ludipress (Lactose, PVP, Crospovidone), Starlac (Lactose, maize starch), Pharmatose DCL (Anhydrous lactose, Guar gum), Avicel CE 15 (MCC, Guar gum), Celocal (MCC, calcium phosphate), Prosolv (MCC, colloidal silica), Di-pac (Sucrose, Dextrin), Advantose FS 95 (Fructose, Starch), Advantose 100 (Maltose), Barcrof CS 90 (Calcium carbonate, starch), Barcrof Premix St (Aluminum hydroxide, Magnesium hydroxide, and Sorbitol), Plasdone S-630 (Vinyl acetate, Vinyl pyrrlidone), Crospovidone, Carbofarma GA10 (Calcium carbonate, Acacia), Carbofarma GM11 (Calcium carbonate, Maltodextrin).

Microcrystalline cellulose is considered as one of the most importantly used ingredient in the pharmaceutical industry formulations of solid dosage forms. This might be due to its wide applicability; it can be used in direct compression and as a diluent in tablets prepared by direct compression, as filler in capsules and for the production of spheres. Microcrystalline cellulose is prepared by partially depolymerization of α-cellulose with mineral acids. It is a white, crystalline powder composed of agglomerated porous microfibers. After purification by filtration and spray drying, porous microcrystals are obtained. It is available in the market under brand names of Avicel, Vivacel, Emcocel, etc. It has different particle sizes; larger grades (Avicel pH 102) had better flowability and lubricity than smaller size grades.

Usually in pharmaceutical solid dosage forms many pharmaceutical excipients are used one of them is the directly compressible filler. These excipients include disintegrants, glidants, lubricants, etc.

Disintegrant is an important additive plays his role after the solid dosage form is ingested is the disintegrant. It is a substance, or a mixture of substances, added to a tablet to facilitate its breakup or disintegration after administration. The active ingredient must be released from the tablet matrix as efficiently as possible to allow for its rapid dissolution. Materials serving as disintegrants have been chemically classified as starches, clays, celluloses, algins, or gums.

The most popular disintegrants are corn and potato starch which have been well-dried and powdered.

In addition to the starches, a large variety of other materials has been used and they are reported to be effective as disintegrants. This group includes Veegum HV, Ac-Di-Sol, methylcellulose, agar, bentonite, cellulose and wood products, natural sponge, cation-exchange resins (e.g., Amberlite IRP-88), alginic acid, guar gum, citrus pulp, and carboxymethylcellulose [D. Gissinger and A. Stamm. Drug Develop. Ind. Pharm., 6, 511 (1980), "A Comparative Evaluation of the Properties of Some Tablet Disintegrants"; E. M. Rudnic, C. T. Rhodes, J. F. Bavitz, and J. B. Schwartz, Drug Develop. Ind. Pharm., 7, 347 (1981), "Some Effects of Relatively Low Levels of Eight Tablet Disintegrants on a Direct Compression System"; H. A. Lieberman and L. Lachman, Pharmaceutical Dosage Forms: Tablets, Volume 1, pages 139-140; and R. E. Gordon, The Thermodynamic Characterization of the Solid Surface Interaction for Three Tablet Disintegrating Agents, Ph.D. Thesis, Purdue University, 1981].

The use of a multifunctional tableting aid with excellent flowability, disintegration and compression properties besides to its self-inertness is a goal of the pharmaceutical excipient industry.

Now a novel mineral-fiber solid-in-solid dispersion technique for manufacturing of directly compressible excipients with surprisingly powerful compression, flow and disintegration properties has been developed and is described in this invention.

### Summary of the invention

The invention refers to a mineral-fiber solid dispersion comprising at least one hydrophilic polymeric material selected from the group consisting of hydrophilic polysaccharides of having the ability to gel in water with minimal dissolution (hydrogel) nature, and at least one silica-based trivalent or divalent salt of a water-absorbable compound selected from the group consisting of silicon dioxide, derivatives thereof or any mixtures thereof.

Moreover, the invention refers to a method for preparing a mineral-fiber solid dispersion via a solid-in-solid dispersion technique by mixing the fiber material with the water-absorbable compound in a presence of water and allowing the absorption of the water-absorbable compound in an appropriate ratio in the intimate structure of the fiber material, and next adjusting the process conditions in order to allow the precipitation of the water-absorbable compound into the mineral solid within the structure of the fiber material to form a solid-in-solid dispersion of mineral and fiber.

The invention is also devoted to use of the mineral-fiber solid dispersion as a pharmaceutical tableting aid for solid dosage forms, and preferably for direct compressible excipient compositions. Liquid dosage forms can also comprise the mineral-fiber solid dispersion as a dispersant material. Also pharmaceutical compositions comprising the mineral-fiber solid dispersion as a tableting aid are covered by this invention.

### Brief description of drawings

Fig. 1 presents the FTIR spectra of (A) sodium silicate, (B) magnesium silicate, (C) chitin, (D) chitin and magnesium silicate physical mixture (1:1 w/w), and (E) chitin-magnesium silicate solid dispersion (1:1 w/w).
Fig. 2 illustrates the tablet hardness [N] of compositions comprising paracetamol and directly compressible materials (chitin-Mg silicate solid dispersion, Avicel 200, Pharmatose DCL21) in different proportions.
Fig. 3 illustrates the tablet hardness [N] of compositions comprising gabapentin and directly compressible materials (chitin-Mg silicate solid dispersion, Avicel 200) in different proportions.
Fig. 4 shows a scheme of the preferred embodiment of the method according to the invention employing the solid-in-solid precipitation technique.

### Detailed description of the invention

In this invention, a pharmaceutical tableting aid is developed based on mineral-fiber solid dispersion technology.

The fibrous materials such as natural hydrophilic polymers suffer relatively of high elasticity and hence less particle-to-particle bonding. The presence of voids within their structures might significantly worsen their mechanical and hardness properties.

Natural hydrophilic polymers namely, polysaccharides are the most abundant organic compounds on the earth. They are linear or branched polymers that are composed of repeating carbohydrate units. They are used by living organisms as food, and also provide structure. Important types of polysaccharides include cellulose, starch, and chitin.

Earth minerals silicates are fragile materials that break rapidly upon compaction, thus when separate they cannot be used in high percentages as pharmaceutical fillers.

The silicates are the largest, the most interesting and the most complicated class of minerals by far. Approximately 30% of all minerals are silicates and some geologists estimate that 90% of the Earth's crust is made up of silicates.

For purposes of the present invention, mineral-fiber solid dispersions are formulated for the preparations as essential constituents comprising minerals such as silicates derivatives or salts, in particular, trivalent and divalent silicates either as single or as mixtures and also, as aggregates of polymeric fillers, e.g. natural or synthetic fibers in particular, hydrophilic natural polymers such as chitin, cellulose, starch, pectin, etc.

To obtain the desired processing property profile of the ready-to-use pharmaceutical tableting aid it is generally necessary to use (in the method) a larger amount of monovalent silicate solution than that required for the subsequent hydration of the fiber material. This is the case particularly when an extremely high compressibility as well as superdisintegration of the ready-to-use pharmaceutical tableting aid is desired. The monovalent silicate is generally absorbed completely inside the fiber particles. The mixture is then dispersed in another solution of trivalent or divalent chloride solution. Next, precipitation of silicates by the di- and trivalent occurs in the fibrous particles, where the solid-in-solid precipitation occurs forming the mineral-fiber solid dispersion.

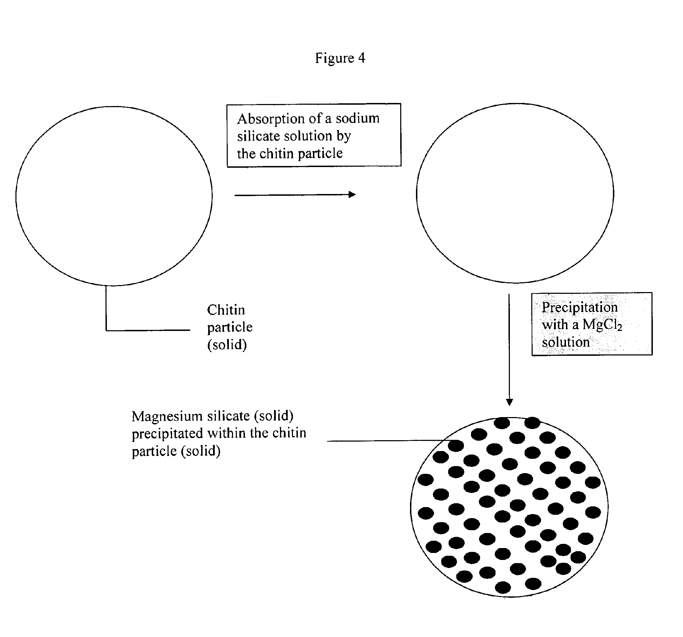

The scheme of the solid-in-solid mineral-fiber dispersion technique on the example of the preferred embodiment is shown in Fig. 4. The hydrogel structure of chitin employed as the fiber material is capable of absorption of water containing sodium silicate in a complete solution state. The particles of chitin saturated with sodium silicate can be immersed in a solution of magnesium chloride. Where, magnesium chloride is capable of diffusing to the inside of the particle. Once magnesium chloride (or any di- or tri-valent chloride salt) enters the particle it will react with sodium silicate and forms an insoluble material called magnesium silicate and a soluble material called sodium chloride. Rinsing of the particles will ensure the elimination of sodium chloride and reserving magnesium silicate as the mineral solid inside the chitin particle. Thus, a solid which is magnesium silicate (Earth mineral) is incorporated inside a solid fiber such as chitin.

In another embodiment of the method according to the invention the solid-in-solid dispersion technique is conducted by mixing a wet mass of the hydrophilic polymeric compound absorbing of the water-absorbable compound in an appropriate ratio in the intimate structure of the polymer and adjusting the process conditions in order to allow the precipitation of the mineral silicate within the structure of the polymer.

This technique is simple in preparation and gives a new pharmaceutical excipient with powerful compaction, disintegration and flow properties.

The ratio of the mineral solid in the mineral-fiber solid dispersion is preferably in the range of 1 to 80% w/w and the most preferable range would be in the range of 20 to 60% w/w. The ratio could be used to modify both the disintegration and compaction properties of the mineral-fiber excipient.

The process of precipitation of a solid mineral silicate in solid fiber particle is carried out under simple mixing procedures. However, any technique known in the art for mixing and drying is also useful such as a fluid bed system for spray granulation or spray drying.

In preferred embodiment of the present invention, the fiber is selected from the group of hydrophilic polymeric materials.

Examples of the hydrophilic polymeric materials include: xanthan gum, chitosan, chitin, sodium alginate, propylene glycol alginate, pectin, starch, guar gum, cellulose, sodium carboxymethyl cellulose, hydroxymethyl propyl cellulose, methyl cellulose, hydroxypropyl cellulose, hydroxyethyl cellulose, cyclodextrins, tragacanth, locust bean gum, carrageenan, polydextrose, dextrin, maltodextrin, polyethylene glycols, polyethylene oxide, polyvinyl alcohol, povidone, etc.

Particularly preferred natural hydrophilic polymeric materials in this embodiment of the invention are cellulose, chitin, starch, pectin and dextrin.

Examples of the silica-based substances include: colloidal silicon dioxide (colloidal silica), magnesium aluminum silicate, magnesium trisilicate, bentonite, kaolin, zinc silicate, iron silicate, calcium silicate, sodium calcium silicate hydroxide, copper silicate hydroxide, sodium aluminum silicate chloride, etc.

In this embodiment, the preferred mineral materials are silicates and in particular trivalent and divalent silicates such as aluminum silicate and magnesium silicate or their combinations

Mineral-fiber solid dispersions can be described as dispersions of one or more mineral material, as a compactable solid material of a relatively high plastic nature, in a fiber or polymer carrier or matrix of a relatively high elastic nature in solid state, which are prepared by enabling the absorption of mineral materials into the fiber or polymer matrix system and the precipitation of the mineral materials within the structure of the fiber solid particles.

Surprisingly it has currently been found that mineral-fiber solid dispersions are most likely a suitable additive for pharmaceutical solid dosage forms, particularly when the trivalent and/or divalent silicates are included within the particle structure of the fiber, particularly the hydrophilic polymer among the abovementioned examples. The mineral-fiber solid dispersion has a powerful compaction behavior besides its rapid disintegration power, and in addition, excellent flowability and inertness of mineral-fiber composition due to the neutral chemical entities. Also, the low cost is a fact of its high availability in nature. These properties of this additive make it a suitable pharmaceutical tableting aid.

In this embodiment of the present invention, the mineral-fiber solid dispersion is selected as a direct compressible excipient with superdisintegration power that can be used in formulation of solid dosage forms in direct compression, wet granulation, and dry granulation formulations.

The mineral-fiber solid dispersion excipient can be mixed with the API separately or with other known in the art pharmaceutical excipients in formulation of solid dosage forms in ratios 1-99% w/w.

The most preferable use of the mineral-fiber solid dispersion is as a pharmaceutical tableting aid in solid dosage forms. However, the inclusion in liquid dosage forms as a dispersant material in formulation of suspensions can be also completed.

### Examples

### Example 1

### Preparation of a mineral-fiber solid dispersion using chitin and Mg silicate and FTIR characteristics thereof

Into 400 ml of deionized water containing 10 g sodium silicate, 10 g of chitin was added and stirred sufficiently at room temperature to form a suspension, and then a solution containing 9.6 g magnesium chloride salt was added to the suspension. Precipitation occurred in the polymer system. The produced wet mass (precipitate) was filtered out and then washed thoroughly with deionized water to get rid of any excess soluble salts. The wet mass was dried at 100°C for 3 hours. The dry mass was sieved on a sieve USP mesh size # 22 then further sieved on a sieve of pore size diameter 425 micrometer. Also a physical mixture of chitin and magnesium silicate with the same proportion was prepared.

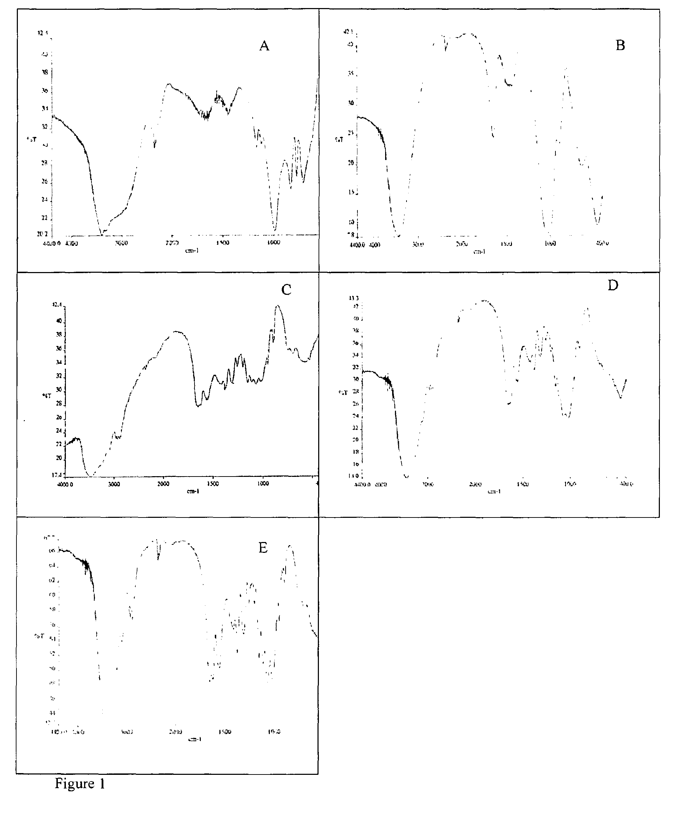

It is obvious that the IR spectra of the chitin-Mg silicate solid dispersion and the physical mixture are not identical, as shown in Fig. 1. They differ dramatically in the sharpness level shown clearly by the solid dispersion when compared to the physical mixture. This may indicate that the solid dispersion has gained high crystalline characteristics that might be responsible for the superior compactibility of the mineral fiber solid dispersion when compared to the physical mixture.

### Example 2

### Preparation of mineral-fiber solid dispersions using chitin as a fiber material and different metal silicates as a mineral material and compaction and disintegration characteristics thereof

Into 400 ml of deionized water containing 10 g sodium silicate, 10 g chitin was added and stirred sufficiently at room temperature to form a suspension, and then a solution containing 9.6 g di- or trivalent metal chloride salt was added to the suspension. Precipitation occurred in the polymer system. The produced wet mass (precipitate) was filtered out and then washed thoroughly with deionized water to get rid of any excess soluble salts. The wet mass was dried at 100°C for 3 hours. The dry mass was sieved on a sieve USP mesh size # 22 then further sieved on a sieve of pore size diameter 425 micrometer. Also physical mixtures of chitin and the corresponding metal silicates with the same proportions and particle sizes were prepared.

600 mg from each type of the chitin-di- or trivalent metal silicate product were compressed into tablets using a manual single press at 1.0 Ton and a punch size of 12 mm shallow concave. 10 repetitions were performed. The following properties were detected:

The prepared mineral-fiber solid dispersion showed superior compaction properties over the physical mixtures as shown in Table 1. Such effectiveness was reflected by the high tablet hardness and low disintegration time. In case of the physical mixtures the disintegration test was not applicable since the tablets had a very low level of hardness (weakly bonded), i.e. disintegrated with mild mechanical stress.

**Table 1: Summary of tablet hardness and disintegration time of the chitin-di- or trivalent metal salt produced in the method of mineral solid dispersion compared to characteristics of the physical mixtures**

| **Polymer type** | **Hardness, N*** | **Hardness, N**** | **Average disintegration time, seconds*** | **Average disintegration time, seconds*** |
|---|---|---|---|---|
| Chitin-Al silicate | >300 | <60 | <2 | NA |
| Chitin-Mg silicate | 250 | <60 | <2 | NA |
| Chitin-Ca silicate | 150 | <60 | <2 | NA |

| | | | | |
|---|---|---|---|---|
| *Mineral-fiber solid dispersion (MFSD) **Physical mixture of the same proportion as MFSD NA: not applicable since the tablet are not good compactable. | | | | |

### Example 3

### Preparation of mineral-fiber solid dispersions using Mg silicate as a mineral material and different hydrophilic polymeric materials as a fiber material

It is desired to extend the method used for the precipitation of metal silicates (magnesium silicate was chosen only as an example) in the presence of chitin and other common hydrophilic polymers used in pharmaceutical industry, such as starch, dextrin, and pectin.

Into 400 ml of deionized water containing 10 g sodium silicate, 10 g polymer either starch, dextrin and pectin was dispersed, and then a solution containing 9.6 g magnesium chloride was added to the dispersion. The produced precipitate was filtered out and then washed with deionized water to remove any excess water soluble salts.

600 mg from each type of the polymer-Mg silicate product were compressed into tablets using a manual single press at 1.0 Ton and a punch size of 12 mm shallow concave. 10 repetitions were performed. The following properties were detected:

Chitin, starch, dextrin, and pectin all showed to be a good example of the effectiveness of the Mg silicate mineral solid dispersions with these polymers. Such effectiveness was reflected by the high tensile strength and low disintegration time of these new modified polymers.

**Table 2: Summary of tablet hardness and disintegration time of combinations of different polymers and magnesium silicate produced in the method of mineral solid dispersion compared to characteristics of the physical mixtures**

| Polymer type | Hardness, N* | Hardness, N** | Average disintegration time, seconds* | Average disintegration time, seconds* |
|---|---|---|---|---|
| Chitin-Mg silicate | 250 | <60 | <2 | NA |
| Starch-Mg silicate | >300 | <60 | 50 | NA |
| Dextrin-Mg silicate | 210 | <50 | 10 | NA |
| Pectin-Mg silicate | 250 | <60 | 10 | NA |

| | | | | |
|---|---|---|---|---|
| *Mineral-fiber solid dispersion (MFSD) **Physical mixture of the same proportion as MFSD NA: not applicable since the tablet is not well bonded. | | | | |

### Example 4

### Effect of dilution with a poorly compactable water-insoluble pharmaceutical active ingredient on mechanical properties of a mineral-fiber solid dispersion (chitin-Mg silicate solid dispersion)

Paracetamol was taken as an example for an active material with poor compaction and poor water-solubility. The active material was mixed in different proportions with the chitin-Mg silicate solid dispersion prior compaction thereof. Next, a constant powder weight i.e. 600 mg of different proportions between the active material and the chitin-Mg silicate solid dispersion were compressed into tablets using a manual single press at 1.0 Ton and a punch size of 13 mm shallow concave. Three repetitions were performed.

For comparison purposes two directly compressible commercial materials (Pharmatose DCL21 and Avicel 200) were also included in the study with using the same previously mentioned procedure.

The directly compressible materials (Avicel 200, Pharmatose DCL21, chitin-Mg silicate solid dispersion) were directly compressed to form slugs that crushed on an Erweka crusher and sieved again on a sieve with a diameter of 200 micrometer, in order to compare same particle sizes, prior to mixing with an active material.

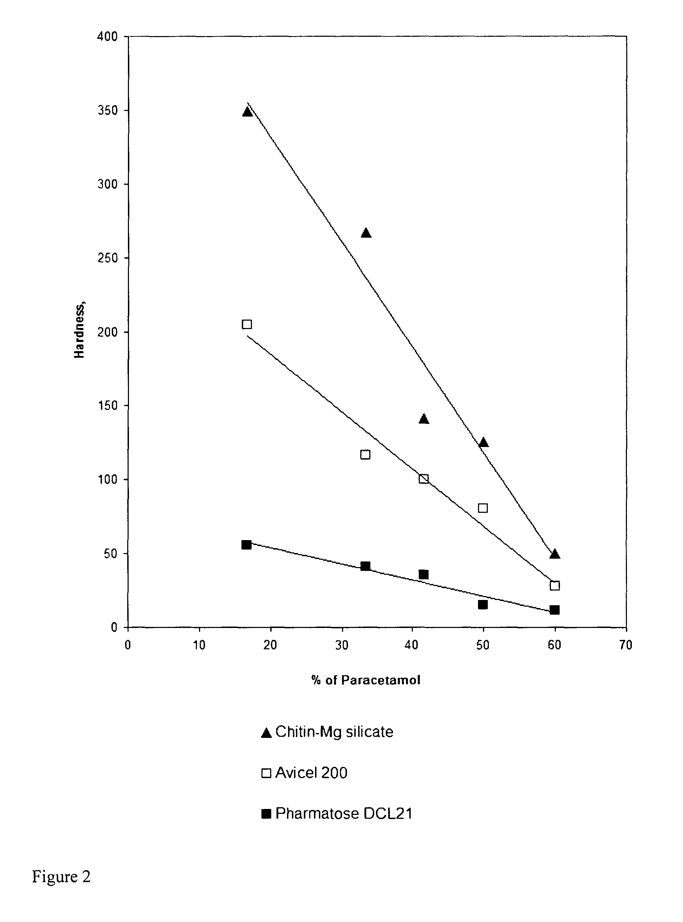

The chitin-Mg silicate solid dispersions showed higher tablet hardness irrespectively to the proportion of the poorly compactable material when compared to Pharmatose DCL21 or Avicel 200 as shown in Fig. 2.

### Example 5

### Effect of dilution with a poorly compactable water-soluble pharmaceutical active ingredient on mechanical properties of a mineral-fiber solid dispersion (chitin-Mg silicate solid dispersion)

Gabapentin was taken as an example for an active material with high water solubility and poor compaction behavior. The active material was mixed in different proportions with the chitin Mg silicate solid dispersion prior compaction thereof. Next, a constant powder weight i.e. 600 mg of different proportions between the active material and the chitin-Mg silicate solid dispersion were compressed into tablets using the manual single press at 1.0 Ton using a punch size of 13 mm shallow concave. Three repetitions were performed.

For comparison purposes Avicel 200 was included in the study with using the same previously mentioned procedure.

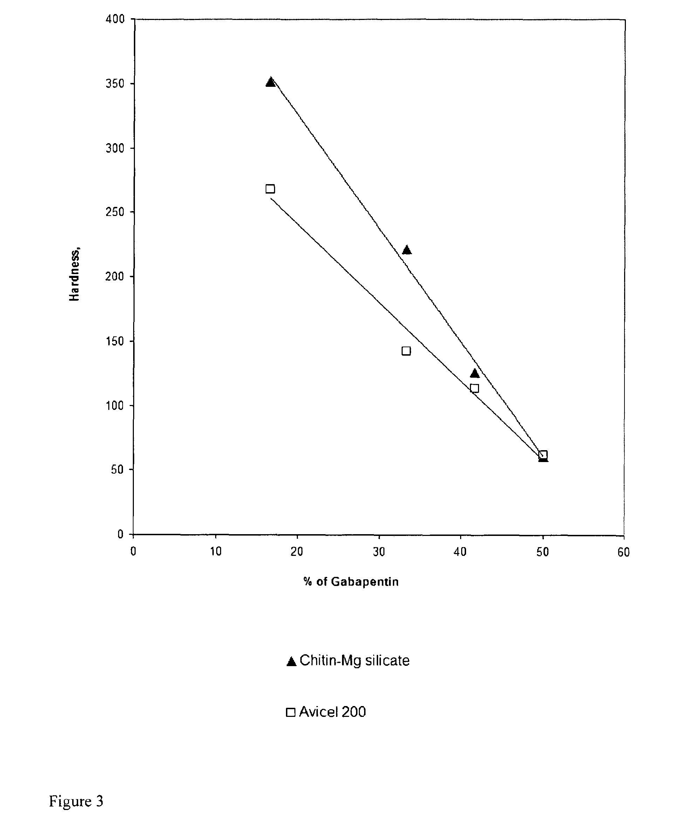

The chitin-Mg silicate solid dispersions showed relatively higher tablet hardness especially at higher polymer concentrations when compared to Avicel 200 as shown in Fig. 3.

The features disclosed in the foregoing description and in the claims may, both separately and in any combination thereof, be material for realizing the invention in diverse forms thereof.

## Claims

1. A mineral-fiber solid dispersion comprising as a fiber material at least one hydrophilic polymeric material preferably selected from the group consisting of hydrophilic polysaccharides having the ability to form a gel in water with minimal dissolution nature, and as a mineral solid at least one silica-based trivalent or divalent salt of a water-absorbable compound selected from the group consisting of silicon dioxide, derivatives thereof or any mixtures thereof.

2. The dispersion according to claim 1, wherein the mineral solid is selected from the group consisting of colloidal silicon dioxide (colloidal silica), magnesium aluminum silicate, magnesium trisilicate, bentonite, kaolin, zinc silicate, iron silicate, calcium silicate, sodium calcium silicate hydroxide, copper silicate hydroxide, and sodium aluminum silicate chloride.

3. The dispersion according to claim 1, wherein the mineral solid is selected from the group of minerals such as silicates, derivatives thereof or salts thereof, or any mixtures thereof.

4. The dispersion according to claim 3, wherein the mineral solid is selected from the group of silicates, preferably trivalent and divalent silicates, either as single or as mixtures thereof.

5. The dispersion according to claim 4, wherein the mineral solid is selected from the group consisting of aluminum silicate, magnesium silicate and their combinations.

6. The dispersion according to any of claims 1 to 5, wherein the fiber material is selected from the group of natural or synthetic hydrophilic polymeric materials such as xanthan gum, chitosan, chitin, sodium alginate, propylene glycol alginate, pectin, starch, guar gum, cellulose, sodium carboxymethyl cellulose, hydroxymethyl propyl cellulose, methyl cellulose, hydroxypropyl cellulose, hydroxyethyl cellulose, cyclodextrins, tragacanth, locust bean gum, carrageenan, polydextrose, dextrin, maltodextrin, polyethylene glycols, polyethylene oxide, polyvinyl alcohol, and povidone.

7. The dispersion according to claim 6, wherein the fiber material is selected from the group of natural hydrophilic polymeric materials such as cellulose, chitin, starch, pectin and dextrin.

8. The dispersion according to any of claims 1 to 7, wherein the ratio of the mineral solid in the mineral-fiber solid dispersion is in the range of 1 to 80% w/w.

9. The dispersion according to claim 8, wherein the ratio of the mineral solid in the mineral-fiber solid dispersion is in the range of 20 to 60% w/w.

10. A method for preparing the mineral-fiber solid dispersion as claimed in any of claims 1 to 9, **characterized by** a solid-in-solid dispersion technique which comprises mixing the fiber material with the water-absorbable compound in a presence of water and allowing the absorption of the water-absorbable compound in an appropriate ratio in the intimate structure of the fiber material, and next adjusting the process conditions in order to allow the precipitation of the water-absorbable compound into the mineral solid within the structure of the fiber material to form a solid-in-solid dispersion of mineral and fiber.

11. An excipient composition comprising the mineral-fiber solid dispersion according to any of claims 1 to 9 as a tableting aid.

12. The excipient composition according to claim 11, further comprising one or more of fillers, disintegrants, glidants, lubricants.

13. The excipient composition according to claims 11 or 12, wherein the composition is a direct compressible excipient composition.

14. A solid dosage form comprising an effective amount of at least one active ingredient and the excipient composition according to any of claims 11 to 13.

15. The solid dosage form according to claim 14, wherein the form is a direct compression formulation.

16. The solid dosage form according to claim 15, wherein the form is a tablet.

17. The solid dosage form according to claim 14, wherein the form is a wet granulation formulation.

18. The solid dosage form according to claim 14, wherein the form is a dry granulation formulation.

19. A liquid dosage form comprising an effective amount of at least one active ingredient and the mineral-fiber solid dispersion according to any of claims 1 to 9 as a dispersant material.

20. A pharmaceutical composition comprising an effective amount of at least one active ingredient and the mineral-fiber solid dispersion according to any of claims 1 to 9 as an excipient.

21. The pharmaceutical composition according to claim 20, further comprising one or more of fillers, disintegrants, glidants, lubricants.

22. The pharmaceutical composition according to claims 20 or 21, comprising poorly compactable water-insoluble pharmaceutical active ingredient.

23. The pharmaceutical composition according to claim 22, comprising paracetamol as the active ingredient.

24. The pharmaceutical composition according to claims 20 or 21, comprising poorly compactable water-soluble pharmaceutical active ingredient.

25. The pharmaceutical composition according to claim 24, comprising gabapentin as the active ingredient.

26. The pharmaceutical composition according to claims 20 to 25 in a form of a tablet.

27. Use of mineral-fiber solid dispersion according to any of claims 1 to 9 as pharmaceutical tableting aid for manufacturing of a solid dosage form.

28. Use of mineral-fiber solid dispersion according to any of claims 1 to 9 as a direct compressible excipient for manufacturing of a solid dosage form.
